(19) Europäisches Patentamt
European Patent Office
Office européen des brevets

(11) Publication number: **0 468 564 A2**

## EUROPEAN PATENT APPLICATION

(21) Application number: **91201723.3**

(22) Date of filing: **04.07.91**

(51) Int. Cl.⁵: **A61K 7/32**, A61K 7/36, A61K 7/48

(30) Priority: **27.07.90 GB 9016525**

(43) Date of publication of application:
**29.01.92 Bulletin 92/05**

(84) Designated Contracting States:
**AT BE CH DE DK ES FR GB GR IT LI NL SE**

(71) Applicant: **UNILEVER NV**
**Burgemeester s'Jacobplein 1 P.O. Box 760**
**NL-3000 DK Rotterdam(NL)**
(84) **BE CH DE DK ES FR GR IT LI NL SE AT**

Applicant: **UNILEVER PLC**
**Unilever House Blackfriars**
**London EC4P 4BO(GB)**
(84) **GB**

(72) Inventor: **Park, Andrew Campbell, Unilever**
**Research**
**Port Sunlight Laboratory, Ouarry Road East**
**Bebington, Wirral, Merseyside L63 3JW(GB)**

(74) Representative: **van Gent, Jan Paulus et al**
**Unilever N.V., Patent Division Postbus 137**
**NL-3130 AC Vlaardingen(NL)**

(54) **Deodorant composition.**

(57) A deodorant composition suitable for topical application to human skin, which comprises:
i an effective amount of a deodorant chosen from salts or oxides of zinc or magnesium, which are sparingly soluble in water;
ii a cross-linked carboxymethylcellulose polymer having a water absorbing capacity of at least 20;
iii a volatile silicone; and
iv a gaseous propellant

EP 0 468 564 A2

FIELD OF THE INVENTION

The invention relates to deodorant compositions suitable for topical application to human skin, particularly liquid compositions suitable for dispensing from a propellant-driven aerosol container.

BACKGROUND TO THE INVENTION

The deodorant and anti-perspirant market is dominated with products based on aluminium or zirconium salts which are intended to prevent, or at least control, perspiration at the skin surface, particularly on the underarm, while simultaneously providing a perceived degree of deodorancy.

It has also been proposed in EP 0 024 176 (Unilever) to employ zinc carbonate in a deodorant aerosol composition for reducing auxiliary body odour without suppressing the secretion of perspiration , the composition comprising a water-absorbent anionic polyelectrolyte. We have now developed an improved approach to the control of perspiration at the skin surface, while maintaining deodorancy, by combining a moisture-absorbent cross-linked cellulosic polymer, which literally mops up perspiration as it appears at the skin's surface, with a volatile silicone to provide superior feel and improved efficacy, and a sparingly soluble zinc or magnesium salt, which not only performs a deodorancy function, but which does not adversely affect the efficacy of this particular moisture-absorbent polymer.

DEFINITION OF THE INVENTION

Accordingly, the invention provides a deodorant composition suitable for topical application to human skin, which comprises:

i. an effective amount of a deodorant chosen from salts or oxides of zinc or magnesium, which are sparingly soluble in water;

ii. a cross-linked carboxymethylcellulose polymer having a water absorbing capacity of at least 20;

iii. a volatile silicone; and

iv. a gaseous propellant.

DISCLOSURE OF THE INVENTION

The Deodorant

The composition according to the invention comprises a deodorant which is chosen from salts or oxides of zinc or magnesium which are insoluble or only sparingly soluble in water.

Examples of suitable salts or oxides are:

zinc oxide
zinc carbonate
zinc citrate
zinc laurate
zinc oleate
zinc orthophosphate
zinc stearate
zinc silicate
zinc tartrate
magnesium oxide
magnesium carbonate
magnesium fluoride
magnesium hydroxide
magnesium laurate
magnesium myristate
magnesium oleate
magnesium palmitate
magnesium stearate
magnesium orthophosphate
magnesium pyrophosphate
magnesium silicate

The amount of deodorant present in the composition according to the invention is from 0.5 to 20%, preferably from 1 to 15% by weight of the composition.

### The Cellulosic Polymer

The composition according to the invention also comprises a cross-linked carboxymethylcellulose polymer having a water absorbing capacity of at least 20.

By "water absorbing capacity of 20" we mean that the polymer has the ability to absorb an amount of water at least 20 times its own weight.

Preferably, the cellulosic polymer will have a water absorbing capacity of at least 30, most preferably of at least 40 or even higher. It is to be understood, however, that at very high water absorbing capacities, e.g. of 100 or more, the polymer will tend to begin to dissolve in the presence of water, and in use can become obtrusive on the skin as it swells and sticky to the touch. Accordingly, the water absorbing capacity should preferably be from 20 to 100.

Particularly preferred examples of the cross-linked carboxymethylcellulose polymer are AKUCELL, available from AKZO, and AQUALON available from HERCULES. The cellulosic polymer should preferably be in a finely divided, powdered form and have an average particle size which is small enough to allow it to pass through the venting orifice of the aerosol container.

The amount of cellulosic polymer present in the composition according to the invention is from 0.5 to 50%, preferably from 1 to 20% by weight of the composition.

### The Volatile Silicone

The composition according to the invention also comprises a linear or cyclic volatile silicone.

Examples of volatile silicones include polydimethyl cyclosiloxane, having a viscosity of less than $5mm^2s^{-1}$, examples of which are DOW CORNING 344 Fluid (tetramer) and DOW CORNING 345 Fluid (pentamer), and volatile hexamethyldisiloxane having a viscosity of not more than $0.65mm^2s^{-1}$, for example DOW CORNING 200 Fluid ($0.65mm^2s^{-1}$).

The preferred volatile silicones are the cyclic forms.

The amount of volatile silicone present in the composition according to the invention is from 1 to 80%, preferably from 5 to 70% by weight of the composition.

### The Propellant

The composition according to the invention also comprises a gaseous propellant to enable to composition to be dispensed from an aerosol container.

Examples of such propellants, which are usually liquefiable, include propane, n-butane, iso-butane and methylene chloride, and these can be used individually or in admixture, with or without other volatile liquids.

Examples of such propellants, which are not usually liquefiable under conditions of normal ambient use include carbon dioxide, nitrogen, nitrogen oxides and air, and these are normally employed in a compressed state in conjunction with a suitable aerosol container.

The amount of gaseous propellant present in the composition according to the invention is from 5 to 95%, preferably from 10 to 80% by weight of the composition.

### Suspending agent

The composition according to the invention can also optionally comprise a suspending agent to assist in suspending the finely divided cellulosic polymer in the composition, to facilitate dispensing the composition in a uniform homogenous state.

Examples of optional suspending agents include hydrophobic clays, such as Bentone-34 and Bentone-38, and colloidal silicas, such as Aerosil 200 and Cab-O-Sil MS, and cellulose derivatives, such as hydroxypropyl cellulose (e.g. Klucel M).

The amount of suspending agent, when present in the composition according to the invention, is usually from 0.5 to 5% by weight of the composition.

### Other Ingredients

The composition according to the invention can comprise other ingredients, in addition to those already identified.

Examples of other ingredients which can optionally be present in the composition according to the invention include:

non-volatile silicones, such as a polydimethylsiloxane having a viscosity in excess of $5mm^2s^{-1}$, for example, from 50 to $1000mm^2s^{-1}$, such as DOW CORNING 200 Fluids (standard viscosities $50-1000mm^2s^{-1}$);

skin feel improves, such as talc and finely divided polyethylene, an example of which is ACUMIST B18;

cosmetically acceptable vehicles, such as anhydrous ethanol and other emollients;

gelling agents, such as stearyl alcohol or waxes, for example Castor wax;

perfumes;

preservatives; and

other cosmetic adjuncts conventionally employed in propellant-driven aerosol deodorant products.

The ingredients other than the deodorant, cellulosic polymer, volatile silicone and propellant, which can optionally be present in the composition according to the invention, can conveniently form the balance of the composition, and accordingly can form up to 90%, preferably from 10 to 80% by weight of the composition.

Product Form

The composition according to the invention can take the form of liquid, containing suspended particulate solids, which is suited to or adapted for topical application to human skin from an aerosol container. The aerosol container can then be used to dispense the composition as a spray to enable it to be applied to the area of the skin, particularly the underarm, where control of perspiration and deodorancy is required.

Use of the composition

The invention also provides for the use of a propellant-driven deodorant composition, in accordance with the invention, as herein defined, in perspiration and body malodour control, following topical application to human skin.

A preferred embodiment of the invention provides for the use of a propellant-driven deodorant composition comprising

i. from 0.5 to 20% by weight of zinc carbonate;

ii. from 0.5 to 50% by weight of cross-linked carboxymethyl cellulose, having a water absorbing capacity of at least 20; and

iii. from 1 to 80% by weight of linear or cyclic volatile silicone; and

iv. from 5 to 95% by weight of a gaseous propellant;

in controlling perspiration and body malodour following topical application to the skin, particularly to the underarm, of the composition according to the invention.

Example

The invention is further illustrated by the following example.

Example 1

This examples illustrates a deodorant aerosol composition according to the invention. The aerosol composition contained the following ingredient:

|  | % w/w |
|---|---|
| Volatile silicone - DOW CORNING 345 | 10.0 |
| Akucell | 2.0 |
| Zinc carbonate | 2.0 |
| Bentone-38 | 0.5 |
| Perfume | 1.0 |
| Propellant (CAP 30*) | 84.5 |

* CAP 30 is a mixture of n-butane, iso-butane and propane.

4

**Claims**

1. A deodorant composition suitable for topical application to human skin, which comprises:

    i. an effective amount of a deodorant chosen from salts or oxides of zinc or magnesium, which are sparingly soluble in water;

    ii. a cross-linked carboxymethylcellulose polymer having a water absorbing capacity of at least 20;

    iii. a volatile silicone; and

    iv. a gaseous propellant.

2. A composition according to claim 1, in which the zinc salt is zinc carbonate.

3. A composition according to claim 1 or 2, in which the deodorant forms from 0.5 to 20% by weight of the composition.

4. A composition according to claim 1, 2 or 3, in which the cellulosic polymer has a water absorbing capacity of at least 30.

5. A composition according to any preceding claim, in which the cellulosic polymer forms from 0.5 to 50% by weight of the composition.

6. A composition according to any preceding claim, in which the volatile silicone is a linear or cyclic polydimethyl siloxane.

7. A composition according to any preceding claim, in which the volatile silicone forms from 1 to 80% by weight of the composition.

8. A composition according to any preceding claim, in which the propellant is chosen from n-butane, iso-butane, propane and mixtures thereof.

9. A composition according to any preceding claim, in which the propellant forms from 5 to 95% by weight of the composition.

10. The use of a propellant-driven deodorant composition comprising:

    i. an effective amount of a deodorant chosen from salts or oxides of zinc or magnesium, which are sparingly soluble in water;

    ii. a cellulose polymer having a water absorbent capacity of at least 20;

    iii. a volatile silicone; and

    iv. a gaseous propellant;

    for the control of perspiration and reduction of body malodour following topical application to human skin.